Europäisches Patentamt

(19) European Patent Office

Office européen des brevets

(11) Numéro de publication: **0 158 543**
**B1**

(12) **FASCICULE DE BREVET EUROPÉEN**

(45) Date de publication du fascicule du brevet:
**23.09.87**

(21) Numéro de dépôt: **85400403.3**

(22) Date de dépôt: **04.03.85**

(51) Int. Cl.⁴: **A 61 F 11/00,** A 61 F 13/20

(54) **Instrument pour le soin des cavités du corps humain et plus particulièrement du conduit auditif.**

(30) Priorité: **05.03.84 FR 8403498**

(43) Date de publication de la demande:
**16.10.85 Bulletin 85/42**

(45) Mention de la délivrance du brevet:
**23.09.87 Bulletin 87/39**

(84) Etats contractants désignés:
**AT BE CH DE FR GB IT LI LU NL SE**

(56) Documents cité:
**EP-A-0 002 904**
**EP-A-0 089 271**
**BE-A-657 615**
**DE-A-2 846 593**
**GB-A-15 173**
**US-A-1 450 612**
**US-A-1 561 927**

(73) Titulaire: **Collin, Marcel, 20, place Lafayette,**
**F-47300 Villeneuve sur Lot (FR)**

(72) Inventeur: **Collin, Marcel, 20, place Lafayette,**
**F-47300 Villeneuve sur Lot (FR)**

(74) Mandataire: **Lordonnois, Michel, B.P. No. 4, F-91230**
**Montgeron Essonne (FR)**

LIBER, STOCKHOLM 1987

## Description

L'invention concerne un nouveau type de tiges, appelées "bâtonnets", instruments pour l'hygiène et les soins des cavités du corps utilisés quelquefois pour débarrasser le nez de ses mucosités, mais plutôt pour extraire le cérumen des oreilles.

Ces tiges, colorées ou non, sont constituées par une baguette en bois, en carton ou en matière plastique, aux extrémités (ou à l'extrémité) de laquelle sont (ou est) fixés des tampons, soit de mousse de plastique, éventuellement de différentes natures, soit de ouate de cellulose, soit de coton hydrophile ou similaire.

On sait que le cérumen, s'il permet la lubrification du conduit auditif externe et arrête les poussières, peut, malheureusement, lorsqu'il s'accumule, former un bouchon qui occasionne de nombreuses gênes, telles que bourdonnements d'oreilles ou une certaine surdité.

On utilisait autrefois, pour enlever ce cérumen, des "cure-oreilles" faits de corne, d'os ou d'ivoire, mais qui comportaient des risques de blessures. Puis, ils ont été remplacés par des bâtonnets munis, à l'une ou à leurs deux extrémités, de coussinets en coton hydrophile, ou en ouate de cellulose de forme ovoïde. Ces coussinets évitent de blesser le conduit et retiennent en partie le cérumen qui adhère à la ouate.

On connaît par exemple dans la technique antérieure divers bâtonnets pourvus d'un tampon de ce genre. C'est le cas, entre autres, dans le brevet US-A-1 450 612 qui décrit un bâonnet pourvu d'un tampon à ailettes, dans le brevet GB-A-15 173 (A.D. 1913) qui d'écrit un tampon plein de forme ovoïde ou tronconique, dans la demande de brevet européen EP-A-0 089 271 qui décrit un tampon de forme ovoïde adapté pour agir à la manière d'une mèche, dans le brevet FR-A-2 249 640 qui décrit un tampon de forme ovoïde et constitué de lamelles souples séparées les unes des autres.

Il est reconnu que le pouvoir d'absorption ou de prise de cérumen de ces tampons ou coussinets est très faible et, qui plus est, ces tampons présentent l'inconvénient, dans la majorité des cas, de repousser le cérumen dans le conduit auditif au lieu de l'extraire, en risquant ainsi de blesser le tympan.

La présente invention a pour but de pallier un tel inconvénient. Elle a donc pour objet un instrument du type bâtonnet constitué par une tige garnie à une ou aux deux extrémités d'un coussinet ou tampon, qui est remarquable en ce qu'il est constitué par un manchon en forme de jupe souple plissée longitudinalement de manière à former des creux et des arêtes parallèles à l'axe longitudinal de ladite tige, manchon qui fait corps avec la tige porteuse centrale et dont l'extrémité libre est évidée sous forme d'une cavité concave, si bien que le cérumen, sous l'enfoncement du

bâtonnet, pénètrera dans cette cavité et dans le creux des plis sans être repoussé et que, suite à une légère rotation du bâtonnet dans n'importe quel sens, il sera décollé du conduit auditif et emprisonné dans les plis. Il est évident que le retrait du bâtonnet occasionnera l'extraction du cérumen ou des matières emprisonnées.

A noter que ces tampons conformes à l'invention, pour faciliter leur pénétration, sont prévus de forme cylindrique ou tronconique, à la manière d'une fraise.

De plus, ils sont constitués en matière relativement souple, telle qu'une composition de fibres papetières mélangées à de la résine (polyester, phénolique), celle-ci étant présente dans une proportion de 5 % environ de la masse totale de la composition.

On peut utiliser également une autre composition, telle qu'un mélange de fibres papetières de Tartas et de fibres de polyéthylène, ces dernières étant présentes dans une proportion de 40 % environ de la masse totale.

D'autres caractéristiques de l'invention apparaitront de la description suivante faite en relation avec les dessins ci-joints, représentant schématiquement, à titre d'exemples, plusieurs modes de réalisation de tampons et dans lesquels:

Les figures 1A et 1B montrent les deux formes générales des tampons conformes à l'invention;

les figures 2A et 2B représentent plus en détails des tampons dont les creux de plis sont de forme concave;

les figures 3A et 3B représentent d'autres modes de réalisation de tampons, dont les plis sont de section trapézoïdale avec creux angulaires.

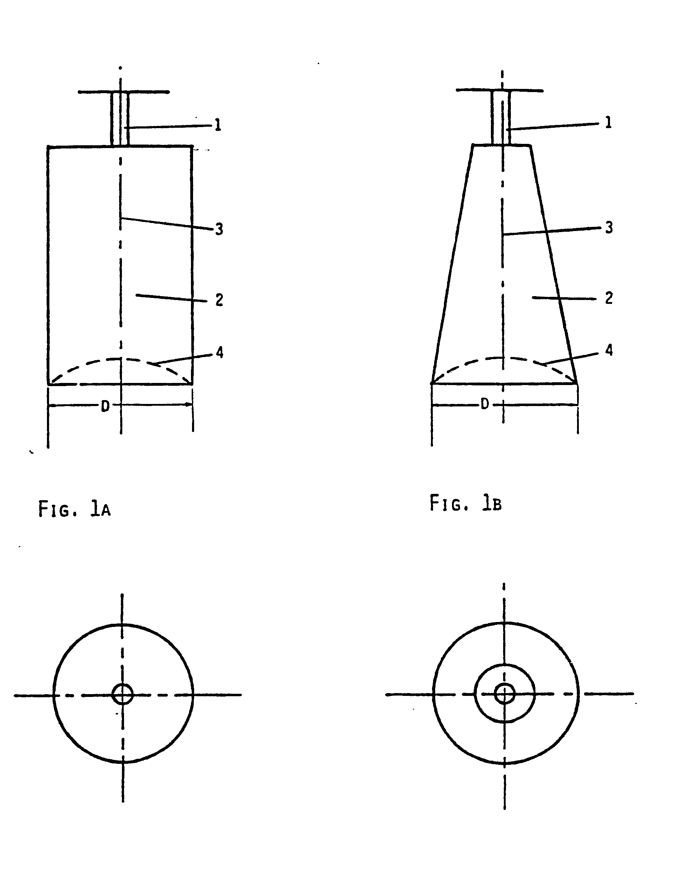

Comme on le remarque des figures 1A et 1B, à l'exemple des instruments connus du type "bâtonnet", l'instrument conforme à l'invention comporte une tige centrale 1 dont l'une et/ou l'autre des extrémités est garnie par un tampon 2 de forme cylindrique ou tronconique de diamètre D et concentrique à l'axe 3 de la tige 1.

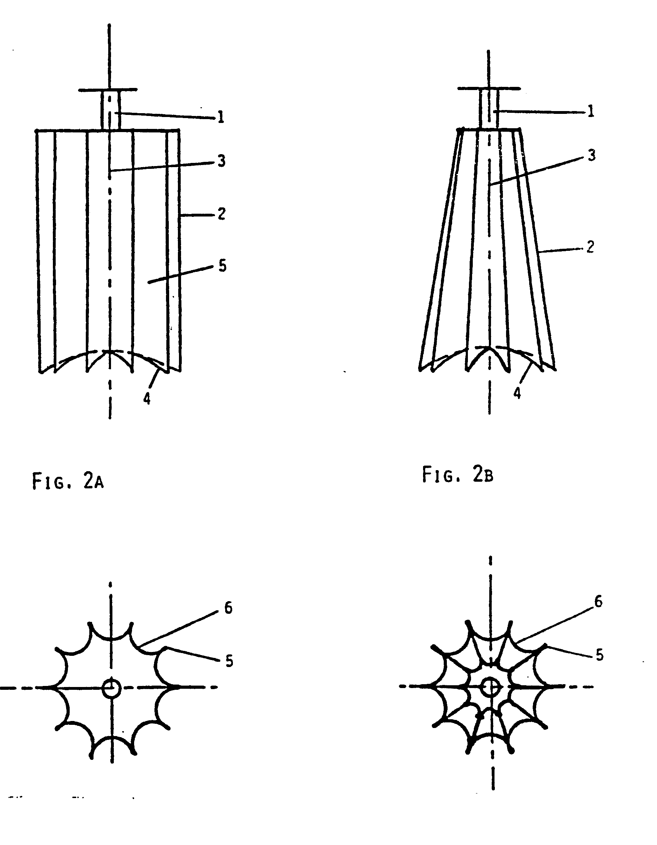

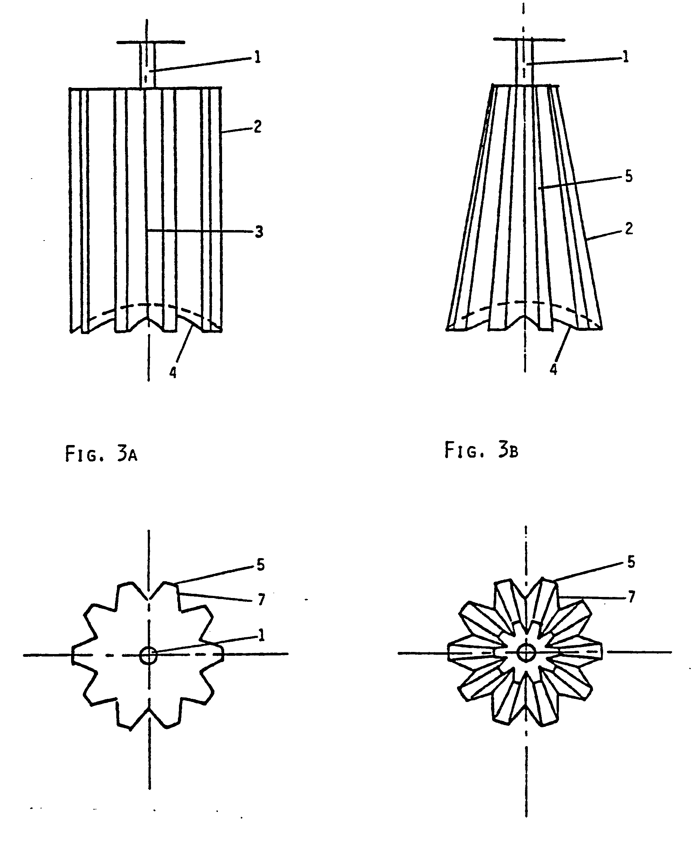

Comme on le remarque des figures 2A, 2B, 3A et 3B, le tampon 2 est constitué par un manchon en forme de jupe plissée souple faisant corps avec la tige porteuse 1 et dont l'extrémité libre 4 ou de pénétration est évidée sous forme d'une cavité concave. Les plis sont adaptés pour avoir des creux 6 de forme concave et des arêtes extérieures angulaires 5 (figures 2A et 2B), ou des creux 7 de forme angulaire et des arêtes 5 extérieures de forme trapézoidale (figures 3A et 3B).

De ces diverses figures il ressort que, dans le cas d'un instrument utilisé par exemple pour enlever le cérumen séjournant dans le conduit auditif, sous l'enfoncement du bâtonnet le cérumen pénétrera dans la cavité concave et dans le creux des plis, sans être repoussé vers le fond du conduit, et que, suite à une légère rotation dans n'importe quel sens du bâtonnet, le cérumen emprisonné, par suite de la souplesse de la matière composant le manchon, se séparera de la masse restante du cérumen.

A noter que la souplesse du manchon permettra l'utilisation de l'instrument dans des conduits auditifs de différents diamètres.

## Revendications

1. Instrument pour l'hygiène et les soins des cavités du corps humain, du type appelé communément "bâtonnet", adapté pour être utilisé plus particulièrement dans l'extraction du cérumen du conduit auditif, cet instrument étant constitué par une tige porteuse centrale (1) garnie à l'une ou à ses deux extrémités par un tampon et caractérisé par le fait que ce tampon est constitué par un manchon (2) en forme de jupe souple plissée longitudinalement de manière à former des creux (6, 7) et des arêtes (5) parallèles à l'axe longitudinal de ladite tige (1), manchon qui fait corps avec la tige porteuse centrale et dont l'extrémité libre (4) ou de pénétration est évidée sous forme d'une cavité concave, si bien que, sous l'enfoncement du bâtonnet dans le conduit auditif, le cérumen pénétrera dans cette cavité et dans le creux (6, 7) des plis sans être repoussé et que, par suite d'une légère rotation du bâtonnet dans n'importe quel sens, le cérumen emprisonné sera dégagé ou détaché de la masse restante et prélevé ou extrait par le retrait du bâtonnet.

2. Instrument selon la revendication 1, caractérisé par le fait que le creux (6) des plis est de forme arrondie alors que l'arête (5) de ceux-ci est de forme angulaire.

3. Instrument selon la revendication 1, caractérisé par le fait que le creux (7) des plis est de forme angulaire et que l'arête (5) de ceux-ci est de forme trapézoidale.

4. Instrument selon la revendication 1, caractérisé par le fait que le manchon plissé (2) est de forme cylindrique droite extérieurement.

5. Instrument selon la revendication 1, caractérisé par le fait que le manchon plissé (2) est de forme tronconique extérieurement, la grande base de cette forme comportant la cavité concave et fournissant l'extrémité libre (4) de pénétration.

6. Instrument selon l'une quelconque des revendications 1 à 5, caractérisé par le fait que la matière souple constituant le tampon ou manchon plissé (2) est une composition de fibres papetières mélangées à de la résine (polyester, phénolique), celle-ci étant présente dans une proportion de 5 à environ de la masse totale de la composition.

7. Instrument selon l'une quelconque des revendications 1 à 5, caractérisé par le fait que la matière souple constituant le tampon ou manchon plissé (2) est une composition telle qu'un mélange de fibres papetières de Tartas et de fibres de polyéthylène, ces dernières étant présentes dans une proportion de 40 % environ de la masse totale.

## Patentansprüche

1. Vorrichtung des allgemein "Stäbchen" genannten Typs zur Pflege von Körperöffnungen und insbesondere zum Entfernen von Ohrenschmalz aus den Gehörgängen, wobei diese Vorrichtung durch einen zentralen Trägerstiel (1) gebildet wird, der an einen Ende oder an seinen beiden Enden mit einem Tampon versehen ist, dadurch gekennzeichnet, daß der Tampon aus einer Muffe (2) in Form eines weichelastischen, in Längsrichtung gefalteten Mantels besteht, um so Vertiefungen (6, 7) und Grate (5) parallel zur Längsachse des Stiels (1) zu bilden, wobei die Muffe mit dem zentralen Tragerstab einstückig ausgebildet und das freie oder Eindringende (4) der Muffe in Form einer konkaven Austiefung ausgehöhlt ist, so daß beim Einführen des Stäbchens in den Gehörgang das Ohrenschmalz in diese Austiefung und in die Vertiefungen (6, 7) der Falten eindringt, ohne zurückgedrückt zu werden, und daß infolge einer leichten Drehung des Stäbchens in irgendeine Richtung das eingefangene Ohrenschmalz von der restlichen Masse freigemacht oder abgelöst und durch Zurückziehen des Stäbchens entnommen oder herausgezogen wird.

2. Vorrichtung nach Anspruch 1, dadurch gekennzeichnet, daß die Vertiefung (6) der Falten eine abgerundete Form aufweist, während ihr Grat (5) winkelförmig ist.

3. Vorrichtung nach Anspruch 1, dadurch gekennzeichnet, daß die Vertiefung (7) der Falten winkelförmig und ihr Grat (5) trapezförmig ist.

4. Vorrichtung nach Anspruch 1, dadurch gekennzeichnet, daß die plissierte Muffe (2) außen die Form eines geraden Zylinders hat.

5. Vorrichtung nach Anspruch 1, dadurch gekennzeichnet, daß die plissierte Muffe (2) außen kegelstumpfförmig ist, wobei die große Grundfläche dieser Form die konkave Höhlung beinhaltet und das freie Eindringende (4) ergibt.

6. Vorrichtung nach einem der Ansprüche 1 bis 5, dadurch gekennzeichnet, daß das weichelastische Material, das den Tampon oder die plissierte Muffe (2) bildet, eine Zusammensetzung von Papierfasern, zugemischt zu (Polyester-, Phenol-)Harz ist, das in einem Verhältnis von ungefähr 5 % der Gesamtmasse der Zusammensetzung vorliegt.

7. Vorrichtung nach einem der Ansprüche 1 bis 5, dadurch gekennzeichnet, daß das weichelastische Material, das den Tampon oder die plissierte Muffe (2) bildet, eine Zusammensetzung, Wie eine Mischung aus Tartaspapierfasern und Polyäthylenfasern ist, wobei die letzteren in einem Verhältnis von ungefähr 40 % der Gesamtmasse vorliegen.

**0 158 543**

## Claims

1. A device for the hygiene and care of orifices of the human body, of the type commonly known as "cotton buds", adapted to be used particularly for the removal of wax from the auditory duct, this device comprising a central carrying stem (1), which has at one or both ends a wad, and characterized in that this wad comprises a flange (2) which is in the form of a pliable skirt pleated longitudinally in a way which creates hollows (6, 7) and ridges (5) which are parallel with the longitidunal axis of the aforesaid stem (1), the flange being integral with the central carrying stem (1), and the free or penetrating end (4) of which is hollowed to form a concave cavity so that when the bud is inserted into the auditory duct, the wax enters into this cavity and into the hollows or insides (6, 7) of the pleats without being pushed back and that following slight rotation of the bud in any direction, the trapped wax is separated from the rest of the wax and is extracted by removal of the bud.

2. A device according to claim 1, characterized in that the inside (6) of the pleat or fold is rounded when the outside or ridge (5) of the pleat is angular.

3. A device according to claim 1, characterized in that the inside (7) of the fold is angular and the ridge (5) of the pleat or fold is trapezoidal.

4. A device according to claim 1, characterized in that the pleated skirt (2) is completely cylindrical.

5. A device according to claim 1, characterized in that the pleated flange (2) is conical, the base of which providing the concave cavity and the free penetrating end.

6. A device according to any of claims 1 to 5, characterized in that the pliable material making up the wad or pleated flange (2) is a combination of paper fibres mixed with a resin (polyester, phenol), the latter being present in a proportion of approximately 5 % of the total mass of the composition.

7. A device according to any of claims 1 to 5, characterized in that the pliant material making up the wad or pleated flange (2) is a combination such as a mixture of paper fibres and of polyethelene fibres, the latter being present in a proportion of approximately 40 % of the total mass.

FIG. 1A

FIG. 1B

FIG. 2A

FIG. 2B

FIG. 3A

FIG. 3B